(19) Europäisches Patentamt
European Patent Office
Office européen des brevets

(11) Publication number: **0 237 125**
**A1**

# (12) EUROPEAN PATENT APPLICATION

(21) Application number: 87200413.0

(22) Date of filing: 06.03.87

(51) Int. Cl.[3]: C 07 C 91/23
C 07 C 93/24, A 61 K 31/135
A 61 K 31/22

(30) Priority: 12.03.86 GB 8606061

(43) Date of publication of application:
16.09.87 Bulletin 87/38

(84) Designated Contracting States:
AT BE CH DE ES FR GB GR IT LI LU NL SE

(71) Applicant: AKZO N.V.
Velperweg 76
NL-6824 BM Arnhem(NL)

(72) Inventor: Rae, Duncan Robertson
17 Gleghorn Road
Lanark Scotland(GB)

(72) Inventor: Anderson, David Millar
Asvale - Riverside Road
Kirkfieldbank Lanark Scotland(GB)

(74) Representative: Hermans, Franciscus G.M. et al,
Postbus 20
NL-5340 BH Oss(NL)

## (54) Novel tricyclic amino compounds.

(57) The present invention relates to novel biologically active tricyclic compounds of the general formula:

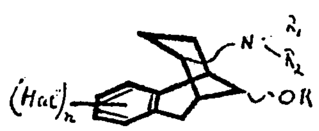

I

and salts thereof
having potent anti-obesity properties.

EP 0 237 125 A1

Croydon Printing Company Ltd.

NOVEL TRICYCLIC AMINO COMPOUNDS

The present invention relates to novel biologically active tricyclic compounds, to processes for the preparation of these compounds and to the pharmaceutical application of these compounds. Particularly the invention relates to novel hexahydro-11-hydroxy-5,9-methanobenzocycloocten-8-amine derivatives of the general formula I:

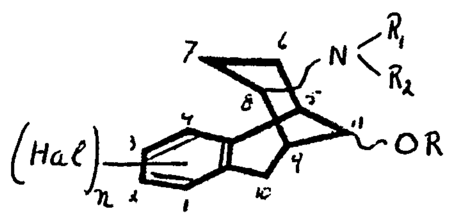

I

and pharmaceutically acceptable salts thereof, in which

$R_1$ and $R_2$ represent the same or different alkyl groups of 1-6 carbon atoms,

R represents hydrogen or an aliphatic acyl group,

Hal means halogen, and

n is 2, 3 or 4.

The compounds of the general formula I have strong anti-obesity properties. This anti-obesity effect is brought about in two ways,

- through reducing food intake, and
- by inhibiting the decline in metabolic rate, which normally accompanies reduced food intake.

Due to the virtually absence of other CNS effects and especially anti-depressants properties which are usually present in analogous compounds, the present compounds qualify for the anti-obesity compounds of choice.

The compounds of the invention can be prepared according to methods in actual use or described in the literature for similar compounds.

For example, the compounds of formula I may be prepared quite conveniently by reduction of the keto group of a compound of the general formula II:

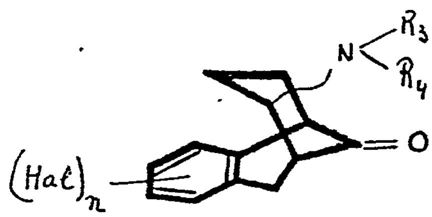

II

or a salt thereof,

in which

n and Hal have the meanings above indicated, and

$R_3$ and $R_4$ represent hydrogen or alkyl (1-6 C).

The reduction is carried out in a way which is usual for the reduction of a keto group into a hydroxyl group, e.g. with the aid of diboran or complex metalhydrides such as $LiALH_4$ or $NaBH_4$.

If $R_3$ and/or $R_4$ are(is) hydrogen in the above synthesis the resulting compound having a primary or secondary amino group should be alkylated in the usual manner into the corresponding compound having a tertiary amino group, for example by reacting the compound (in which $R_3$ and/or $R_4$ = hydrogen) with an alkyl halide or by acylating the compound followed by reduction of the carbonyl group.

Another method for preparing the compounds of the invention consists of the reduction of the azide of the formula III:

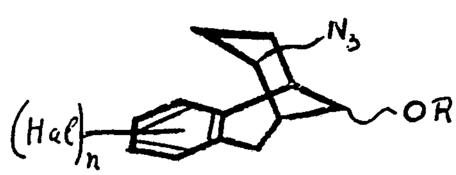

III

in which R, n and Hal have the meanings above indicated.

The reduction is carried out in a manner which is well known in the reduction of azides for example with the aid of $LiALH_4$, or by catalytic hydrogenation with the aid of e.g. $H_2/PtO_2$ or $H_2/Pt$.

The primary amino compound thus obtained is subsequently alkylated in the usual manner, for example by reacting the compound with alkyl halide or by acylation followed by reduction of the carbonyl moiety.

For the introduction of methyl groups at the nitrogen atom the method of Eschweiler-Clarke or the reaction with formaldehyde or haloformic esters, followed by reduction is to be preferred.

The starting compounds of formula II and III can be prepared along the general lines described in the literature. A flow sheet indicating one of the possible methods for the preparation of these starting compounds II and III is attached to this specification.

Where a compound I has been obtained, in which R is hydrogen, the compound may additionally be acylated to obtain a compound in which R is an acyl group. This acylation may be carried out by one of the usual reaction methods for the preparation of an ester, for example by reaction of the alcohol of formula I with the appropriate acid halide or acid anhydride.

The compounds of formula I possess four asymetric centres. As a result thereof a compound I may consist of a mixture of various racemic diastereo-isomers. These racemic diastereo-isomers may be separated, if desired, by physico-chemical techniques such as fractional crystallisation, column chromatography, preparative thin-layer chromatography or counter current distribution.

The extensive separation, however, should preferably be avoided by carrying out the separation of diastereo-isomers already in an earlier stage of the total synthesis.

For example, the compound of formula II or of formula IV consists of two racemic diastereo-isomers, viz. one racemic isomer in which the hydroxyl group (formula IV) or the amino group (formula II) is in exo

position and the other racemic isomer having that substituent in endo position. Separation of the mixture of diastereo-isomers at this stage of the synthesis results in only one racemic diastereo-isomer of the formula II or IV, in which the substituent (hydroxyl or amino) is either in exo or in endo position.

The subsequent reduction of the keto group at position 11 introduces a further asymetric center, which may result in further diastereo-isomers which, of course, can be separated; the reduction of the keto group, however, often turns out to be almost stereospecific, especially if the amino-moiety at position 8 is in exo-position.

A racemic diastereo-isomer of formula I may further be resolved in the usual manner for the resolution of an optically active acid such as (+) or (-) tartaric acid. The resolution may, of course, also be carried out in an earlier stage of the synthesis.

The racemic as well as the optically active diastereo-isomers are numbered among the compounds of the invention. The preferred stereo chemical configuration, however, is the racemic or optically active diastereo-isomer of formula I in which the amino moiety at position 8 is in exo position and the hydroxyl moiety (or acyloxy moiety) at position 11 in anti position.

The novel compounds of formula I may be isolated from the reaction mixture in the form of a pharmaceutically acceptable salt, dependent on the conditions in which the reaction is carried out. The pharmaceutically acceptable salts may also be obtained by treating the free base I with an organic or inorganic acid such as HCl, HBr, HI, $H_2SO_4$, $H_3PO_4$, acetic acid, propionic acid, glycolic acid, maleic acid, malonic acid, succinic acid, tartaric acid, citric acid, benzoic acid, ascorbic acid etc.

The term "alkyl" used in the definition of $R_1$ and $R_2$ means a saturated branched or unbranched hydrocarbon radical with 1-6 carbon atoms, such as methyl, ethyl, n.propyl, n.butyl, isopropyl, isobutyl, t.butyl, n-pentyl, isopentyl and hexyl. Preferably, both moieties $R_1$ and $R_2$ are the same, and in particular methyl or ethyl.

By halogen is preferably meant a chloro- or bromo-group. Preferably the halogen substituents at the benzo moiety of the compounds I are the same.
The preferred substitution pattern is the dihalo substitution (hence n = 2) with a special preference for the halogen substituents in adjacent positions.
The most preferred substitution pattern at the benzo moiety is the 2,3-dihalo and, in particular the 2,3-dichloro-substitution pattern.

An "acyl group" mentioned in the definition of R means a group derived from an aliphatic carboxylic acid. The aliphatic carboxylic acids cover acids with 1-18 carbon atoms, including a carbocyclic ring, and particularly those with 1-8 carbon atoms, such as acetic acid, propionic acid, butyric acid, iso-butyric acid, valeric acid, hexanoic acid, heptanoic acid, trimethyl acetic acid, cyclopentanecarboxylic acid and cyclohexanecarboxylic acid. The lower aliphatic acyl group with 1-4 carbon atoms is the most preferred acyl group.

The compounds of the invention may be administered enterally or parenterally, preferably in a daily dosage of from 0.01 - 10 mg per kg bodyweight.

Mixed with suitable auxiliaries the compounds I may be compressed into solid dosage units, such as pills, tablets and coated tablets or be processed into capsules.

By means of suitable liquids the compounds I can also be applied as an injection preparation in the form of solutions, suspensions or emulsions.

Preferred compounds according to the invention have the general formula:

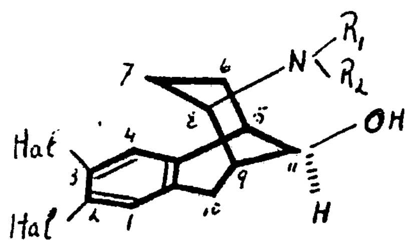

and salts thereof,
in which
$R_1$ and $R_2$ represent alkyl (1-4 C) and more preferably both methyl or ethyl, the amino moiety at position 8 is in exo position and the hydroxyl moiety at position 11 in anti position.

Example 1

(5α,8α,9α,11R*)-8-amino-2,3-dichloro-5,6,7,8,9,10-hexahydro-5,9-methanobenzocycloocten-11-ol

A solution of (5α,8α,9α,11R*)-8-azido-2,3-dichloro-5,6,7,8,9,10-hexahydro-5,9-methanobenzocycloocten-11-ol (5 g) in dichloromethane (130 ml) and ethanol (130 ml) was shaken in the presence of platinum oxide (50 mg), in an atmosphere of hydrogen at 45 psi for 4 h. The solution was filtered and evaporated and the product was crystallised from dichloromethane/methanol to give the amino alcohol (4.7 g) m.p. 206-208 °C.

Example 2

(5α,8α,9α,11R*)-2,3-dichloro-8-dimethylamino-5,6,7,8,9,10-hexahydro-5,9-methanobenzocycloocten-11-ol hydrochloride

An aqueous solution of sodium borohydride (1 g) was added to a solution of (5α,8α,9α)-2,3-dichloro-8-dimethylamino-5,6,7,8,9,10-hexahydro-5,9-methanobenzocycloocten-11-one (5 g) in methanol (5) ml) and the solution was stirred at room temperature for 3 h. Water was added to the solution and the product was extracted with ether. The extract was washed with water, dried with sodium sulphate and evaporated to give the dimethylamino alcohol as a gum (5 g). Hydrogen chloride was passed into an ethereal solution of the amino alcohol and the resultant precipitate was crystallised from methanol to give the hydrochloride salt (4.5 g) m.p. 280 ° (decomp.).

## Example 3

(5α,8α,9α,11R*)-2,3-dichloro-8-dimethylamino-5,6,7,8,9,10-hexahydro-5,9-methanobenzocycloocten-11-ol hydrochloride

A mixture of the amino alcohol (8.2 g) formic acid (16 ml) and formalin (16 ml) was heated on a steam bath for 25 h. The solution was cooled, poured into water and the solution was basified. The product was extracted with ether and the extract was washed with water, dried with sodium sulphate and evaporated to give the dimethyl amino alcohol as a gum (8.6 g). The product was dissolved in ether, hydrogen chloride gas was added and the precipitated product was collected and crystallised from methanol to give the hydrochloride salt m.p. 280 ° (decomp.).

## Example 4

In an analogous manner as described in Example 1 and 3 is prepared:
(5α,8α,9α,11R*)-1,2,3-trichloro-8-dimethylamino-5,6,7,8,9,10-hexahydro-5,9-methanobenzocycloocten-11-ol.HCl.

## Example 5

Acetic anhydride (0.2 ml) was added to a solution of the dimethylamino alcohol of Example 3 (1 g) in pyridine (5 ml) and the solution was allowed to stand at room temperature overnight. Water was added to the solution which was then basified and subsequently extracted with ether. The extract was washed well with water, dried with sodium sulphate, and evaporated to give the acetyl derivative as an oil. The oil was dissolved in ether, HCl-gas was added and the precipitate was crystallised from methanol to give the HCl salt of the acetyl derivative. M.p. = 280-284 °C.

CLAIMS

1. A compound of the formula:

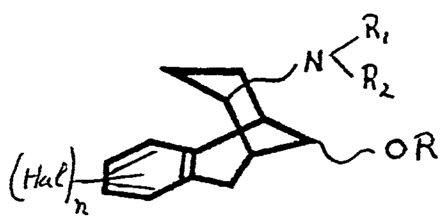

or a pharmaceutically acceptable salt thereof,

in which

$R_1$ and $R_2$ represent the same or different alkyl groups of 1-6 carbon atoms,

R represents hydrogen or an aliphatic acyl group,

Hal means halogen, and

n is 2, 3 or 4.

2. A compound according to claim 1 of the formula:

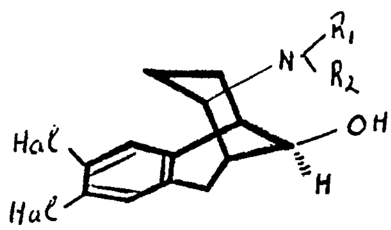

or a salt thereof,
in which
$R_1$ and $R_2$ represent alkyl (1-4 C) and more preferably both methyl or ethyl, the amino moiety at position 8 is in exo position and the hydroxyl moiety at position 11 in anti position.

3. A pharmaceutical composition containing the compound of claim 1 or 2 in admixture with the usual pharmaceutical carriers.

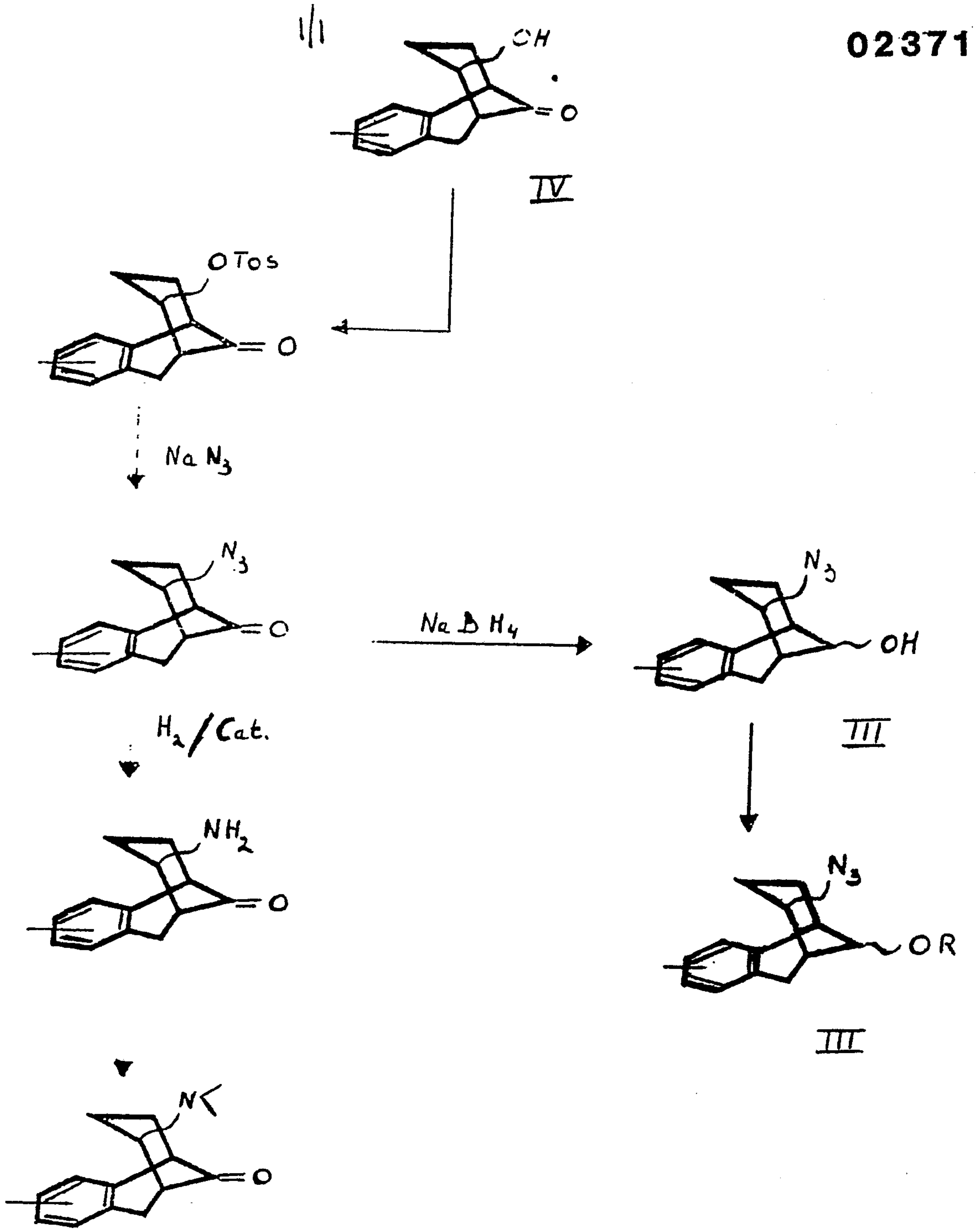


IV

III

III

II

European Patent Office

# EUROPEAN SEARCH REPORT

0237125 Application number

EP 87200413.0

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| A | CH - A5 - 632 985 (AKZO N.V.)<br>* Page 5, right column, lines 3, 4; page 3, left column, lines 34-37 *<br>-- | 1-3 | C 07 C 91/23<br>C 07 C 93/24<br>A 61 K 31/135<br>A 61 K 31/22 |
| A | CH - A5 - 628 612 (AKZO N.V.)<br>* Examples 11,13; page 3, right column, lines 59-62 *<br>-- | 1-3 | |
| A | US - A - 4 008 277 (HEWETT et al.)<br>* Abstract; column 13, lines 7,8 *<br>---- | 1,3 | |
| | | | TECHNICAL FIELDS SEARCHED (Int. Cl.4) |
| | | | C 07 C 91/00<br>C 07 C 93/00 |

The present search report has been drawn up for all claims

| Place of search | Date of completion of the search | Examiner |
|---|---|---|
| VIENNA | 26-05-1987 | KÖRBER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document